# EUROPEAN PATENT SPECIFICATION

(11) **EP 2 799 050 B1**
(45) Date of publication and mention of the grant of the patent: **19.07.2017**
(21) Application number: 12862232.1
(22) Date of filing: 28.12.2012
(51) Int. Cl.: A61F 13/49, A61F 13/514

(54) **DISPOSABLE ABSORPTIVE ARTICLE**
SAUGFÄHIGER WEGWERFARTIKEL
ARTICLE ABSORBANT JETABLE

(30) Priority: 28.12.2011 JP 2011289429
(43) Date of publication of application: 05.11.2014
(73) Proprietor: Unicharm Corporation, Ehime 799-0111 (JP)
(72) Inventor: TODA, Haruki, Kanonji-shi Kagawa 769-1602 (JP); KINOSHITA, Akiyoshi, Kanonji-shi Kagawa 769-1602 (JP)
(74) Representative: Murgatroyd, Susan Elizabeth
(86) International application number: PCT/JP2012/084224
(87) International publication number: WO 2013/100151

(56) References cited:
- EP-A1- 2 537 496
- WO-A1-98/20822
- JP-A- 2007 282 829
- JP-A- 2011 167 416
- JP-U- 3 171 928

## Description

### (Technical Field)

The present invention relates to disposable urine pads having means for prevention of bedsore.

### {Background}

Conventionally, absorbent articles having means for prevention of bedsore is well known. For example, the Patent Literature 1 discloses a diaper including a topsheet, a backsheet and an absorbent body lying between these sheets. The absorbent body is a laminate structure formed from a plurality of laminated absorbent cores adapted to be movable relative to one another in a shear direction. Relative movability of these absorbent cores in the shear direction makes it possible to prevent unacceptable frictional resistance from being generated between a skin-contactable surface of the diaper and a wearer's skin. Sometimes it is required to turn up part of the bed in order to set up an upper half of a bedridden wearer's body so that this part may be utilized as a backrest for the wearer. In the course of partially turning up the bed in this manner, the wearer's body slides downward along with the high friction and the high shearing force generated between the wearer' s skin and the diaper. Considering that the shearing force generated between the wearer's skin and the diaper is one of causes for the bedsore, the generation of such shearing force may be restricted to prevent the bedsore from developing.

### {Citation List}

### {Patent Literature}

{PTL 1}: JP 2008-183159 A

### {Summary}

### {Technical Problem}

The above-cited PTL 1 certainly discloses a relationship between the wearer's skin and the absorbent article but discloses nothing about a relationship between the absorbent article and wearer's garment put on the outer side of the article. For example, a frictional resistance between the absorbent article and the wearer's garment may be reduced to make relative movements of the absorbent article and the garment smooth, thereby restricting the displacement of the absorbent article relative to the wearer's skin. In consequence, the shearing force between the wearer's skin and the absorbent article may be restricted to prevent the development of the bedsore. However, PTL 1 teaches nothing about this viewpoint. In this regard, PTL 1 teaches nothing also about a package suitable for the absorbent articles.

JP 2007 - 282829 discloses a sanitary napkin including a liquid-permeable topsheet, a liquid-impermeable backsheet and an absorbent body disposed between the sheets. The skin-facing side of the napkin has a rear adhesive portion for securing to the skin and the non-skin facing side of the rear part of the napkin has a processing unit so as to be slippery with respect to the shorts of the wearer. The napkin provides a good fit and leakage prevention is improved.

An object of the present invention is to provide a disposable urine pad improved so that a frictional resistance of the non-skin-contactable surface of the urine pad may be reduced.

### (Solution to Problem)

A first aspect of the present invention relates to an improvement of a disposable urine pad having a longitudinal direction and a transverse direction and including a skin-contactable surface, a non-skin-contactable surface extending on the opposite side of the skin-contactable surface, an imaginary transverse center line bisecting a dimension of the urine pad in the longitudinal center line, an imaginary longitudinal center line bisecting a dimension of the urine pad in the transverse direction, front and rear end edges extending in the transverse direction, a topsheet lying on the skin-contactable surface, a backsheet lying on the non-skin-contactable surface and an absorbent body lying between the top- and backsheets, the absorbent body including a liquid-absorbent core material, and the non-skin-contactable surface being formed with a low-friction region having a frictional resistance smaller than in the other regions and the low-friction region extending between the imaginary transverse center line and a rear end region.

In the disposable urine pad as described above, the first aspect of the present invention is characterized in that the absorbent body includes a wearer's body pressure buffering region defined between the imaginary transverse center line and the rear end edge in which a dimension in the thickness direction of the core material is smaller than in the remaining regions so that the low-friction region may overlap the wearer's body pressure buffering region and the low-friction region extends outwards in the transverse direction beyond both sides of the wearer's body pressure buffering region.

In second and third aspects of the present invention, there is included a package of the disposable urine pads each folded along one or more fold lines extending in the transverse direction to form two or more layers in a thickness direction thereof and put into a packaging bag.

The second aspect of the present invention is characterized in that the disposable urine pad includes first and second fold lines extending in the transverse direction and spaced apart from each other in the longitudinal direction, a rear region defined between the rear end edge and the first fold line, an intermediate region defined between the first and second fold lines, a front region defined between the second fold line and the front end edge and the low-friction region lying on the non-skin-contactable surface of the rear region and having a frictional resistance smaller than in the backsheet and wherein the rear region is folded along the second fold line onto the intermediate region so that the skin-contactable surface of the rear region may face the skin-contactable surface of the intermediate region and the front region is folded along the first fold line onto the non-skin-contactable surface of the rear region so as to cover the low-friction region.

The third aspect of the present invention is characterized in that the disposable urine pad includes first and second fold lines extending in the transverse direction and spaced apart from each other in the longitudinal direction, a rear region defined between the rear end edge and the first fold line, an intermediate region defined between the first and second fold lines, a front region defined between the second fold line and the front end edge and the low-friction region lying on the non-skin-contactable surface of the rear region and having a frictional resistance smaller than in the backsheet and wherein the front region is folded along the second fold line onto the intermediate region so that the skin-contactable surface of the front region faces the skin-contactable surface of the intermediate region and the rear region is folded along the first fold line onto the non-skin-contactable surface of the front region so as to expose the low-friction region on the outside.

### {Advantageous Effects of Invention}

According to the first aspect of the present invention, the disposable urine pad is formed on the non-skin-contactable surface thereof with the low-friction region having the frictional resistance smaller than in the remaining regions, this low-friction region is defined on the side of rear end edge with respect to the imaginary transverse center line. With such arrangement, slipperiness of the disposable urine pad relative to the clothes or the like lying outside the article is ensured to prevent undesirable shearing force from being generated between the disposable urine pad and the wearer's skin. In consequence, it is possible to prevent the bedsore from developing due to the shearing force generated between the wearer's skin and the disposable urine pad.

According to the second aspect of the present invention, the disposable urine pad may be folded with the low-friction region unexposed on the outside to make a handling to stack a plurality of the disposable urine pads.

According to the third aspect of the present invention, the individual disposable urine pad may be folded with the respective low-friction regions exposed on the outside to make it possible to take out the article smoothly one by one from the packaging bag in which a plurality of the disposable urine pads have been put into the packaging bag under compression.

### {Brief Description of Drawings}

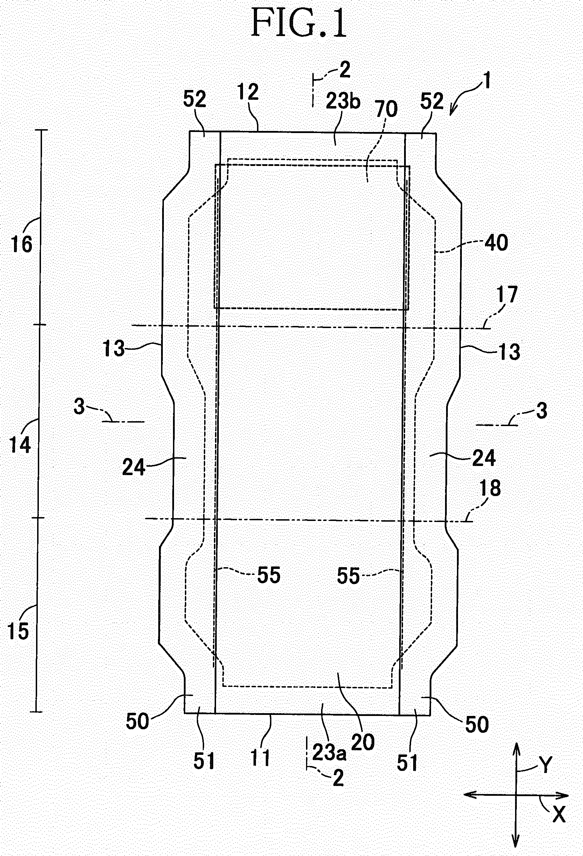

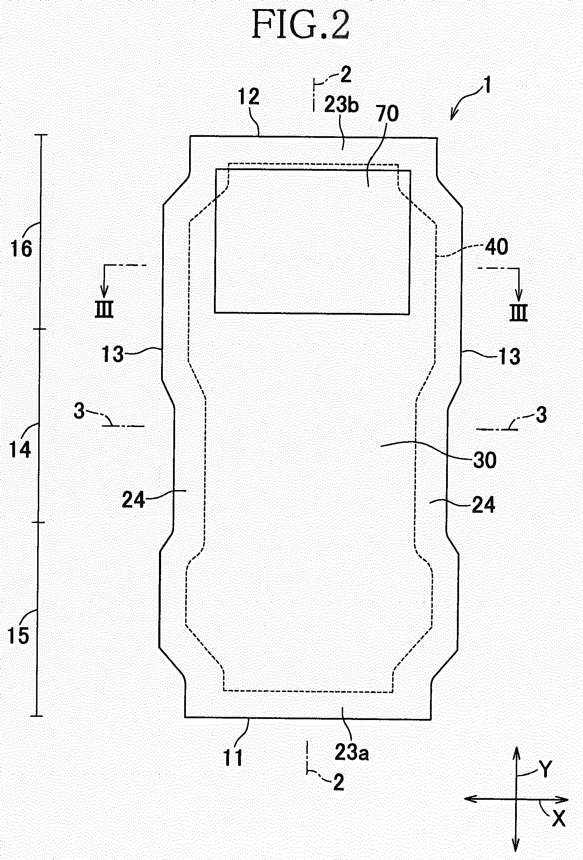

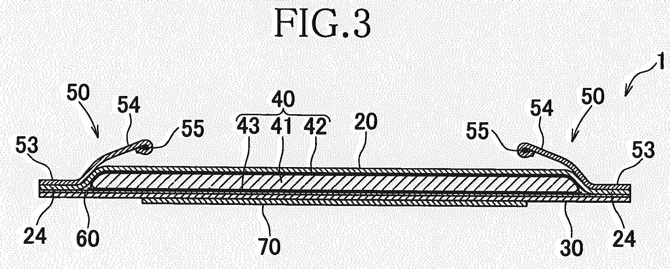

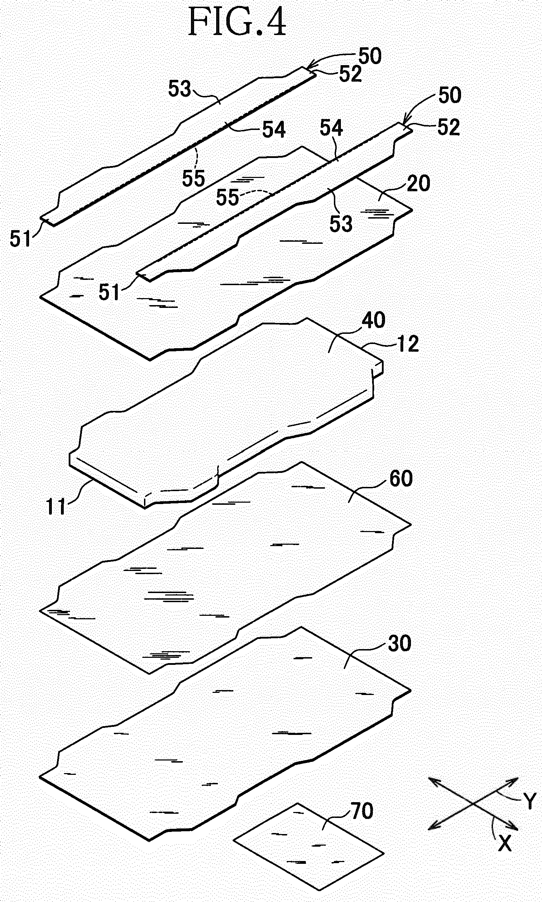

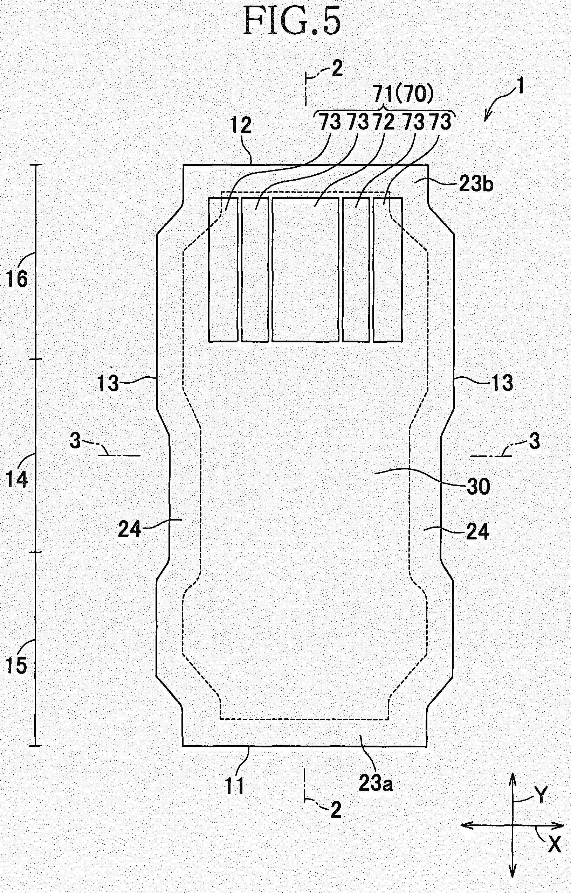

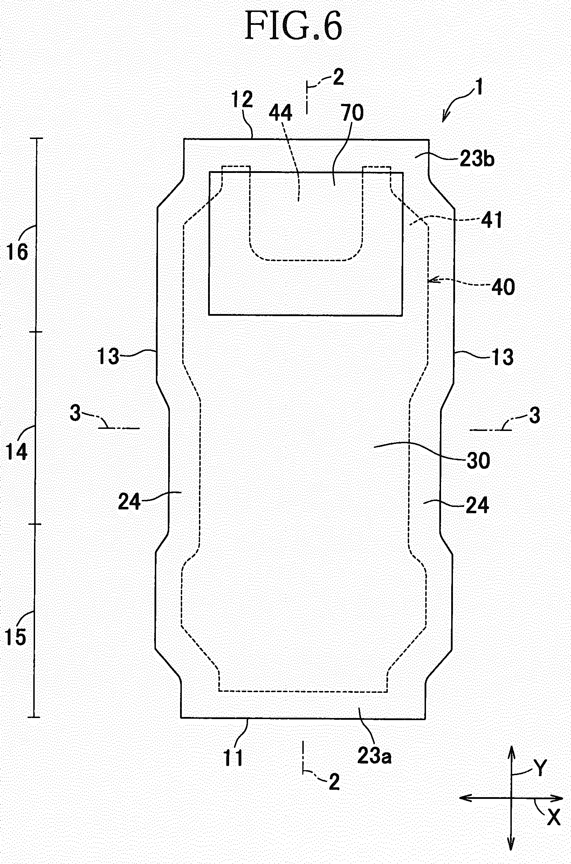

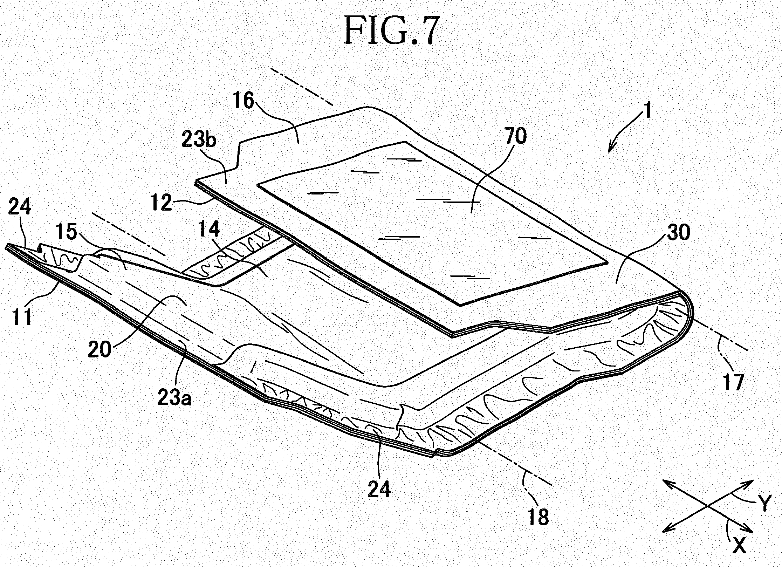

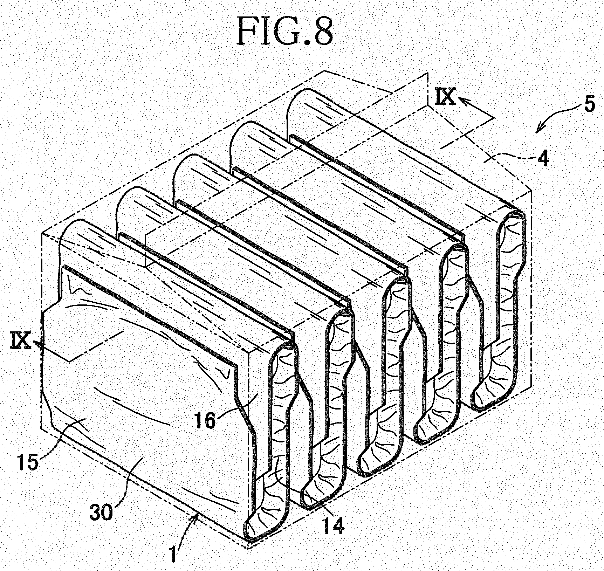

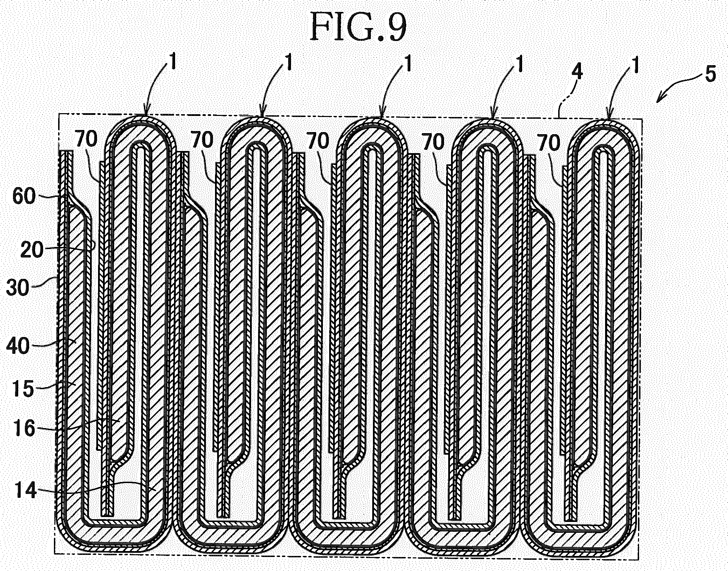

{Fig. 1} Fig. 1 is a plan view of an arrangement of a disposable urine pad included for background information as viewed from a skin-contactable surface.
{Fig. 2} Fig. 2 is a plan view of the urine pad as viewed from a non-skin-contactable surface.
{Fig. 3} Fig. 3 is a scale-enlarged sectional view taken along line III-III in Fig. 2.
{Fig. 4} Fig. 4 is an exploded perspective view of the urine pad.
{Fig. 5} Fig. 5 is a plan view of another arrangement of the urine pad included for background information as viewed from the non-skin-contactable surface.
{Fig. 6} Fig.6 is a plan view of an embodiment of the urine pad as viewed from the non-skin-contactable surface.
{Fig. 7} Fig. 7 is a perspective view illustrating a process of folding the urine pad.
{Fig. 8} Fig. 8 is a perspective view of a package containing therein a plurality of the urine pads.
{Fig. 9} Fig. 9 is a scale-enlarged sectional view taken along line IX-IX.

### {Description}

Referring to Figs. 1 and 2, a urine pad 1 has a longitudinal direction Y and a transverse direction X and includes front and rear end edges 11, 12 extending in the transverse direction X, both side edges 13 extending in the longitudinal direction Y. The urine pad 1 has an imaginary longitudinal center line 2-2 bisecting a dimension in the transverse direction X and an imaginary transverse center line 3-3 bisecting a dimension in the longitudinal direction X wherein the urine pad 1 is symmetric about the imaginary longitudinal center line 2-2. The pad 1 is approximately trisected into an intermediate region 14, a front region 15 and a rear region 16.

Referring to Figs. 2 through Fig. 4, the urine pad 1 includes a liquid-permeable and hydrophilic topsheet 20 lying on the skin-contactable surface, a hardly liquid permeable or liquid-impermeable backsheet 30 lying on the non-skin-contactable surface and an absorbent body 40 lying between these sheets 20, 30. The top- and backsheets 20, 30 extend outward beyond the both end edges so as to form a pair of end flaps 23a, 23b and extend outward beyond the side edges of the absorbent body 40 so as to form a pair of side flaps 24. A leakage-barrier sheet 60 is arranged between the absorbent body 40 and the backsheet 30 and a pair of containment sheets 50 extending in in the longitudinal direction Y and spaced apart from each other in the transverse direction X is disposed to the topsheet 20.

As material for the topsheet 20, for example, spunbond fibrous nonwoven fabrics, point-bond fibrous nonwoven fabrics or point-bond fibrous nonwoven fabrics having a mass per unit area in a range of about 15 to 35 g/m2 may be used. As material for the backsheet 30, for example, spunbond/meltblown/spunbond (designated hereunder as SMS) fibrous nonwoven fabrics, spunbond fibrous nonwoven fabrics or laminates composed of at least one of these fibrous nonwoven fabrics and plastic films, each having a mass per unit area in a range of about 10 to 30 g/m2 may be used. As the leakage-barrier sheet 60, air-permeable and liquid-impermeable plastic films may be used.

As material for the containment sheets 5, for example, SMS fibrous nonwoven fabrics having a mass per unit area in a range of 10 to 30 g/m2 may be used. Each of the containment sheets 50 extends between the front end edge 11 and the rear end edge 12 in the longitudinal direction Y and has front and rear end edges 51, 52, a proximal side edge 53 attached to the topsheet 30 and a distal side edge 54 being free to be spaced away upward from the topsheet 10. Along the distal side edge 54 of the containment sheet 50 a sleeve or a loop are formed and an elastic member 55 extending in the longitudinal direction Y is contractibly attached under tension within the sleeve or loop. With the article put on a wearer's body, the distal side edge 54 are spaced away upward from the topsheet 20 under contractile force of the elastic members 55 in close contact with the wearer' thighs to contain body exudates within the containment sheets 50.

The absorbent body 40 includes a liquid-absorbent core material 41 and liquid-diffusible wrapping sheets 42, 43 such as tissue paper adapted to cover an absorbing surface and a bottom surface (See Fig. 3). As the core material 41, the absorbing material widely used for the absorbent article of this type, specifically, wood fluff pulp, superabsorbent polymer particles or mixture thereof may be used.

A dimension of the absorbent body 40 in the transverse direction X within a predetermined region thereof defined in the vicinity of the imaginary transverse center line 3-3 is set to be smaller than dimensions in the transverse direction X of the front and rear end regions 11,12. In this embodiment , the region dimensioned in the transverse direction X to be relatively small is displaced with respect to the imaginary transverse center line 3-3 towards the front region so that a larger amount of body exudates may be absorbed more quickly in the rear region than in the front region. Consequently, even when the wearer discharges urine in a supine posture, the urine can be reliably absorbed without an anxiety that urine might leak out of the urine pad 1.

The urine pad 1 is formed on the non-skin-contactable surface thereof with a low-friction region 70 in which the frictional resistance is lower than that of the backsheet 30. The low-friction region 70 is allocated between the imaginary transverse center line 3-3 and the rear end edge 12, more preferably in an entirety of the rear end region 16 (See Fig. 2). The low-friction region 70 includes a base material sheet and a silicon resin distributed thereon. As the base material sheet, a plastic film formed of a thermoplastic resin such as polyethylene or polypropylene may be used and this film may be coated with silicone resin having a mass per unit area in a range of about 0.6 to about 0.8 g/my. As the silicon resin, solvent-type, solventless-type or emulsion-type silicon resin may be used so far as the type-classification is concerned and a thermosetting or ultraviolet-curing silicon resin may be used so far as the setting or curing behavior classification is concerned. In this regard, the silicon resin of the ultraviolet-curing type is preferably used for the reason that, if the thermosetting plastic film is used as the base material sheet, there is a possibility that the base material sheet might be molten when the base material sheet is heated in order to cure the silicon resin.

In this arrangement, a static friction coefficient was about 0.31 and a dynamic friction coefficient was about 0.25 in the low-friction region 70. A static friction coefficient was about 0.63 and a dynamic friction coefficient was about 0.49 on the backsheet 30.

### <Measurement of frictional coefficient>

As the friction coefficients, the static friction coefficient and the dynamic friction coefficient were measured. The static friction coefficient and the dynamic friction coefficient were measured with the use of Horizontal Method of JIS P8147 (b). As a test piece for a horizontal strip, frictional cotton cloth "union cloth No. 3 (shirting No. 3)" prescribed in JIS L0803 was used and, as a test piece for a weight, a silicon-coated sheet and the backsheet respectively placed in the low friction region 70 were used.

A friction coefficient in the low-friction region 70 may be adjusted so as to be smaller than in the other regions to assure that the urine pad 1 becomes sufficiently slippery along clothes or the like covering the urine pad 1 from the outer side to move smoothly relative to the clothes or the like. Even when the wearer slides down along the backrest of the bed, the urine pad 1 moves together with the wearer's skin smoothly relative to the clothes so that a shear force between the urine pad and the wearer's skin may be minimized and whereby the wearer may be protected against the development of bedsore. In this regard, it is possible to set the urine pad 1 on the inner side of a diaper, thereby using the urine pad 1 in the form of combination with the diaper. It is also possible to set the urine pad 1 on the inner side of clothes such as an undergarment, thereby using the urine pad 1 in the form of combination with the undergarment.

If the low-friction region 70 is formed over an entirety of the backsheet 30, a quantity of material to be used will necessarily increases and cause a manufacturing cost to be correspondingly increased. However, the backsheet 30 may be partially formed with the low-friction region 70 to avoid such increased cost. From another viewpoint, if the low-friction region 70 is formed over an entirety of the backsheet 30, not only stiffness of the backsheet 30 will be increased but also air-permeability thereof also will be deteriorated and vapor stuffiness within the urine pad will be apprehended. The backsheet 30 may be partially formed with the low-friction region 70 to avoid such problems. Furthermore, allowing that the low-friction region 70 is formed over the entirety of the backsheet 30, slipperiness of the urine pad relative to the undergarment or the diaper will be improved. However, particularly after urination, the intermediate region 14 and the vicinity thereof will be subject to an excessive load and the slipperiness of the urine pad 1 relative to the undergarment or the diaper also will become excessive. The backsheet 30 may be partially formed with the low-friction region 70 to avoid this problem also.

The urine pad 1 as has been described above maybe effectively used, for example, particularly for bedridden aged persons. The bedridden aged person often has his or her sacral bone abnormally protruding due to various aging phenomena such as muscular degeneration and the vicinity of such protruding sacral bone is contact with the urine pad 1 more tightly than the remaining body regions. In consequence, there is a possibility that undesirable shearing force might be generated between the region in the vicinity of the sacral bone and the urine pad 1 and develop the bedsore. Particularly when part of the bed is turned up to set up an upper half of a bedridden wearer's body or the bedridden wearer is transferred from the bed onto a wheel chair, there is a high possibility that a shearing force might be generated between the vicinity of the sacral bone and the urine pad. However, the low-friction region 70 may be arranged in the rear region 16 so as to overlap the vicinity of the sacral bone to prevent effectively a shearing force from being generated between the vicinity of the sacral bone and the urine pad 1.

Fig. 5 illustrates another arrangement of the low-friction region 70. The low-friction region 70 includes a plurality of elongate sub-regions 71 extending in the longitudinal direction Y and discontinuously arranged in the transverse direction X. The elongate sub-regions 71 include a central sub-region 72 on the imaginary longitudinal center line 2-2 and a plurality of lateral sub-regions 73 arranged on both sides of the central sub-region 72 so that each of the adjacent sub-regions 71 may be spaced from each other. The central sub-region 72 has a dimension in the transverse direction X is larger than that of the respective lateral sub-regions 73. More specifically, a dimension in the transverse direction X of the central sub-region 72 may be about 120 mm, a dimension in the transverse direction X of the respective lateral sub-regions 73 may be about 25 mm. A distance dimension between the central sub-region 72 and the adjacent lateral sub-regions 73 and a distance dimension between each pair of the adjacent lateral sub-regions 73 may be about 10 mm. A plurality of the elongate sub-regions 71 may be arranged discontinuously in the transverse direction X in this manner to reduce material and manufacturing costs. In addition, each pair of the adjacent elongate sub-regions may be spaced from each other to ensure the air-permeability of the urine pad 1.

While the base sheet coated with silicon resin is used as the low-friction region 70 according to this embodiment, it is also possible to coat a region of the backsheet 30 corresponding to the low-friction region 70 directly with a silicon resin. When a fibrous nonwoven fabric is used as the backsheet 30, the backsheet 30 may be coated with a silicon resin at a mass per unit area in a range of about 1.0 to about 1.5 g/m2. While any one of the conventional methods such as spraying method, a rolling method and a dipping method may be selectively used as the coating method, the spraying method or the rollingmethodmay be preferably used from the viewpoint that only the non-skin-contactable surface is coated with a silicon resin. In this regard, the resin with which the backsheet is coated is not limited to a silicon resin and it is also possible to use a fluorine resin for the similar effect.

The base sheet of the low-friction region 70 is bonded to the backsheet 30 with the use of well known bonding means such as hot melt adhesive. The bonding means may be continuously or discontinuously distributed in the longitudinal direction Y and the transverse direction X. The bonding means may be discontinuously distributed to ensure the air-permeability and thereby to prevent vapor stuffiness within the urine pad 1. The backsheet 30 and the leakage-barrier sheet 60 may be bonded to each other with the use of well known bonding means such as hot melt adhesive (not shown) and such bonding means also may be continuously or discontinuously distributed. For example, when the base sheet of the low-friction region 70 and the backsheet 30 are bonded to each other through the intermediary of the continuously distributed bonding means, at least in the region corresponding thereto, the backsheet 30 and the leakage-barrier sheet 60 may be bonded to each other preferably through the intermediary of the discontinuously distributed bonding means. Either bonding means may be discontinuously distributed in this manner to ensure the air-permeability.

Fig. 6 illustrates an embodiment of the urine pad 1. This embodiment is characterized in that the absorbent body 40 is partially cut out to define a wearer's body pressure buffering region 44. According to this embodiment, the wearer's body pressure buffering region 44 is defined by a sub-region of the rear region 16, in which the core material 41 of the absorbent body 40 is not present. The wearer's body pressure buffering region 44 is located on the imaginary longitudinal center line 2-2 and the core material 41 is present in both laterals in the transverse direction X of this wearer's body pressure buffering region 44. The low-friction region 70 overlaps the wearer's body pressure buffering region 44 and extends outward in the transverse direction X beyond both sides of the wearer's body pressure buffering region 44. It is essential for the wearer' s body pressure buffering region 44 to have a dimension in the thickness direction of the core material 41 is smaller than in the remaining regions. To meet this essential requirement, the core material may be partially cutaway to define a region in which substantially no core material 41 is present or a mass per unit area in the given region may be adjusted to be lower than in the remaining regions.

The urine pad 1 described above is put on an aged person's body so that the vicinity of the sacral bone may be aligned with the body pressure buffering region 44. By putting the urine pad 1 on the wearer's body in this manner, it is possible to prevent the vicinity of the wearer's sacral bone from being put in a tight contact with the urine pad 1, thereby preventing more reliably the bedsore from developing in the vicinity of the sacral bone. In this regard, it is also possible to form the low-friction region 70 so as to be coincident only with the wearer's body pressure buffering region 44 or to extend further outward in the transverse direction X as well as in the longitudinal direction Y beyond the wearer's body pressure buffering region 44.

It is possible to form the low-friction region 70 from a plurality of the elongate sub-regions discontinuously arranged in the transverse direction X. Here it is also possible to arrange these sub-regions in a manner that the central sub-region 72 may overlap the wearer's body pressure buffering region 44 and the lateral sub-regions 73 may overlap the core material 41 on both lateral portions of the wearer's body pressure buffering region 44.

Figs. 7 through 9 illustrate a manner in which the urine pad 1 described above is packaged. Referring to Fig. 7, the urine pad 1 has first and second fold lines 17, 18 extending in the transverse direction X and spaced apart from each other in the longitudinal direction Y. The first and second fold lines 17, 18 coincide with lines trisecting a dimension in the longitudinal direction Y of the urine pad 1 wherein the first fold line 17 is located on the side of the rear end edge 12 and the second fold line 18 is located on the side of the front end edge 11. The urine pad 1 is folded along the first fold line 17 so that the topsheet 20 in the intermediate region 14 and the rear region 16 may face each other and the front region 15 is folded along the second fold line 18 so as to cover the backsheet 30 of the rear region 16. In this way, the urine pad 1 is folded in three so that the front region 15 may be exposed on the outside and the rear region 16 may be laid on the inside thereof. In consequence, the low-friction region 70 arranged on the rear region 16 is laid on the inside of the front region 15 and not exposed on the outside in the urine pad 1 folded in three.

Referring to Figs. 8 and 9, a plurality of the urine pads 1 each folded in three are stacked in the thickness direction and put into a packaging bag 4 to make an individual package. When a predetermined number of the urine pads have been stacked, these urine pads are held and compressed between a pair of arms (not shown) in a packaging system and put into a packaging bag 4 to form a package 5. The urine pad 1 is folded in three in the manner that the low-friction region 70 is not exposed on the outside and consequently these urine pads 1 are put into the packaging bag 4 in orderly stacked state even when the urine pads 1 are held and compressed by the arms. If the urine pads 1 are folded in the manner that the respective low-friction regions 70 are exposed on the outside, the urine pads 1 will slip along the low-friction regions 70 of the respective urine pads 1 stacked one on another when they are hold and compressed by the arms and there is a possibility that these urine pads 1 could not be orderly put into the packaging bag 4.

However, the urine pad 1 may be folded in three in the manner that the low-friction region 70 of the urine pad 1 is exposed on the outside when the urine pads 1 are put into the packaging bag 4 one by one and then stacked within the packaging bag 4. When the low-friction regions 70 are exposed on the outside as in this case, the urine pad 1 can be smoothly taken out from the packaging bag 4. In contrast, if the respective folded urine pads 1 are packaged under compression with the respective low-friction regions 70 unexposed on the outside, it is rather difficult to take out the urine pads 1 smoothly one by one from the packaging bag 4. This is for the reason that when it is tried to take out one urine pad 1 from the packaging bag, the adjacent urine pad 1 is apt to accompany the former. However, the urine pads 1 may be stacked by the intermediary of the low-friction regions to facilitate the urine pads 1 to be taken out one by one from the packaging bag 4.

The disclosure relating to the present invention described above may be arranged at least as follows:
A first aspect of the present invention: A disposable absorbent article 1 having a longitudinal direction Y and a transverse direction X and including a skin-contactable surface, a non-skin-contactable surface extending on an opposite side of the skin-contactable surface, an imaginary transverse center line 3-3 bisecting a dimension of the absorbent article 1 in the longitudinal direction Y, an imaginary longitudinal direction Y bisecting a dimension of the absorbent article 1 in the transverse direction X, front and rear end edges 11, 12 extending in the transverse direction X, a topsheet 20 lying on the skin-contactable surface, a backsheet 30 lying on the non-skin-contactable surface and an absorbent body 40 lying between the top- and backsheets 20, 30.

The first aspect of the present invention further includes:
the non-skin-contactable surface is formed with a low-friction region 70 having a frictional resistance smaller than in the other regions and the low-friction region 70 extends between the imaginary transverse center line 3-3 and the rear end region 12, and the absorbent body 40 includes a liquid-absorbent core material 41 and a wearer's body pressure buffering region 44 defined between the imaginary transverse center line 3-3 and the rear end edge 12 in which a dimension in the thickness direction of the core material 41 is smaller than in the remaining regions so that the low-friction region 70 may overlap the wearer's body pressure buffering region 44.

The first aspect of the present invention may include at least embodiments as described below:
(1) The low-friction region 70 includes at least one of a silicon resin and a fluorine resin.
(2) The low-friction region 70 overlaps the absorbent body 40.
(3) The low-friction region 70 is composed of a plurality of elongate sub-regions 71 extending in the longitudinal direction Y and discontinuously arranged in the transverse direction X.
(4) The low-friction region 70 includes a base sheet and a silicon resin distributed on the base sheet.

The second aspect of the present invention: The disposable absorbent article includes first and second fold lines 17, 18 extending in the transverse direction X and spaced apart from each other in the longitudinal direction X, a rear region 16 defined between the rear end edge 12 and the first fold line 17, an intermediate region 14 defined between the first and second fold lines 17, 16, a front region 15 defined between the second fold line 18 and the front end edge 11 and the low-friction region 70 lying on the non-skin-contactable surface of the rear region 16 and having a frictional resistance smaller than in the backsheet 30 and wherein the rear region 16 is folded along the second fold line 17 onto the intermediate region 14 so that the skin-contactable surface of the rear region 16 faces the skin-contactable surface of the intermediate region 14 and the front region 15 is folded along the first fold line 18 onto the non-skin-contactable surface of the rear region 16 so as to cover the low-friction region 70.

The third aspect of the present invention: The disposable absorbent article includes first and second fold lines 17, 18 extending in the transverse direction X and spaced apart from each other in the longitudinal direction X, a rear region 16 defined between the rear end edge 12 and the first fold line 17, an intermediate region 14 defined between the first and second fold lines 17, 16, a front region 15 defined between the second fold line 18 and the front end edge 11 and the low-friction region 70 lying on the non-skin-contactable surface of the rear region 16 and having a frictional resistance smaller than in the backsheet 30 and wherein the front region 15 is folded along the second fold line 18 onto the intermediate region 14 so that the skin-contactable surface of the front region 15 faces the skin-contactable surface of the intermediate region 14 and the rear region 16 is folded along the first fold line 17 onto the non-skin-contactable surface of the front region 15 so as to expose the low-friction region 70 on the outside.

The constituent elements of the disposable urine pad 1 as one embodiment of the disposable absorbent article are not limited to those described in the specification but the other various well known types of material widely used in the relevant technical field may be used without limitation unless otherwise stated. Terms "first" and "second" used in the specification of this invention are used merely to distinguish the similar elements, similar positions or the other similar means.

### {Reference Signs List}

- 1: urine pad (disposable absorbent article)
- 2-2: imaginary longitudinal center line
- 3-3: imaginary transverse center line
- 4: packaging bag
- 5: packaged articles
- 11: front end edge
- 12: rear end edge
- 14: intermediate region
- 15: front region
- 16: rear region
- 17: first fold line
- 18: second fold line
- 20: topsheet
- 30: backsheet
- 40: absorbent body
- 41: core material
- 44: wearer' s body pressure buffering region
- 70: low-friction region
- 71: elongate sub-regions
- X: transverse direction
- Y: longitudinal direction

## Claims

1. A disposable urine pad (1) having a longitudinal direction (Y) and a transverse direction (X) and including a skin-contactable surface, a non-skin-contactable surface extending on opposite side of the skin-contactable surface, an imaginary transverse center line (3-3) bisecting a dimension of the urine pad in the longitudinal center line (2-2), an imaginary longitudinal direction (Y) bisecting a dimension of the urine pad (1) in the transverse direction (X), front and rear end edges (11,12) extending in the transverse direction (X), a topsheet (20) lying on the skin-contactable surface, a backsheet (30) lying on the non-skin-contactable surface and an absorbent body (40) lying between the top- and backsheets (20, 30), the absorbent body (40) including a liquid-absorbent core material (41), and the non-skin-contactable surface being formed with a low-friction region (70) having a frictional resistance smaller than in the other regions and the low-friction region (70) extending between the imaginary transverse center line (3-3) and a rear end region (16);
**characterized in that**:
the absorbent body (40) includes a wearer's body pressure buffering region (44) defined between the imaginary transverse center line (3-3) and the rear end edge (12) in which a dimension in the thickness direction of the core material (41) is smaller than in the remaining regions so that the low-friction region (70) may overlap the wearer's body pressure buffering region (44) and the low-friction region (70) extends outwards in the transverse direction (X) beyond both sides of the wearer's body pressure buffering region (44).

2. The disposable urine pad (1) according to claim 1, wherein the low-friction region (70) includes at least one of a silicon resin and a fluorine resin.

3. The disposable urine pad (1) according to claim 1 or 2, wherein the low-friction region (70) is composed of a plurality of elongate sub-regions (71) extending in the longitudinal direction (Y) and discontinuously arranged in the transverse direction (X).

4. The disposable urine pad (1) according to any one of claims 1 through 3, wherein the low-friction region (70) includes a base sheet and a silicon resin distributed on the base sheet.

5. The disposable urine pad (1) according to any one of claims 1 through 4, **characterized in that** the disposable urine pad includes first and second fold lines (17,18) extending in the transverse direction (X) and spaced apart from each other in the longitudinal direction (Y), a rear region (16) defined between the rear end edge (12) and the first fold line (17), an intermediate region (14) defined between the first and second fold lines (17,18), a front region (15) defined between the second fold line (18) and the front end edge (11) and the low-friction region (70) lying on the non-skin-contactable surface of the rear region (16) and having a frictional resistance smaller than in the backsheet (30) wherein the rear region (16) is folded along the second fold line (17) onto the intermediate region (14) so that the skin-contactable surface of the rear region (16) faces the skin-contactable surface of the intermediate region (14) and the front region (15) is folded along the first fold line (18) onto the non-skin-contactable surface of the rear region (16) so as to cover the low-friction region (70).

6. The disposable urine pad (1) according to any one of claims 1 through 4, **characterized in that** the disposable urine pad (1) includes first and second fold lines (17,18) extending in the transverse direction (X) and spaced from each other in the longitudinal direction (Y), a rear region (16) defined between the rear end edge (12) and the first fold line (17), an intermediate region (14) defined between the first and second fold lines (17,18), a front region (15) defined between the second fold line (18) and the front end edge (11) and the low-friction region (70) lying on the non-skin-contactable surface of the rear region (16) and having a frictional resistance smaller than in the backsheet (30) and wherein the front region (15) is folded along the second fold line (18) onto the intermediate region (14) so that the skin-contactable surface of the front region (15) faces the skin-contactable surface of the intermediate region (14) and the rear region (16) is folded along the first fold line (17) onto the non-skin-contactable surface of the front region (15) so as to expose the low-friction region (70) on the outside.

## Patentansprüche

1. Einweg-Urinkissen (1), das eine Längsrichtung (Y) und eine Querrichtung (X) hat und eine die Haut kontaktierende Oberfläche, eine die Haut nicht kontaktierende Oberfläche, die sich auf der gegenüberliegenden Seite der die Haut kontaktierenden Oberfläche erstreckt, eine imaginäre Quer-Mittellinie (3-3), die eine Abmessung des Urinkissens in der LängsMittellinie (2-2) zweiteilt, eine imaginäre Längsrichtung (Y), welche eine Abmessung des Urinkissens (1) in der Querrichtung (X) zweiteilt, eine vordere und eine hintere Stirnkante (11, 12), die sich in der Querrichtung (X) erstrecken, eine Deckschicht (20), die auf der die Haut kontaktierenden Oberfläche liegt, eine Rückschicht (30), die auf der die Haut nicht kontaktierenden Oberfläche liegt, und einen absorbierenden Körper (40) aufweist, der zwischen der Deck- und der Rückschicht (20, 30) liegt, wobei der absorbierende Körper (40) ein flüssigkeitsabsorbierendes Material (41) aufweist, und die die Haut nicht kontaktierende Oberfläche mit einem reibungsarmen Bereich (70) ausgebildet ist, der einen Reibungswiderstand hat, der kleiner als der in den anderen Bereichen ist, und der reibungsarme Bereich (70) sich zwischen der imaginären Quer-Mittellinie (3-3) und dem hinteren Stirnbereich (16) erstreckt;
**dadurch gekennzeichnet, dass**
der absorbierende Körper (40) einen gegenüber einem Körper des Trägers druckabfedernden Bereich (44) aufweist, der zwischen der imaginären Mittellinie (3-3) und der hinteren Stirnkante (12) definiert ist, bei dem eine Abmessung in der Dickenrichtung des Kernmaterials (41) kleiner als in den übrigen Bereichen ist, sodass sich der reibungsarme Bereich (70) mit dem gegenüber dem Körper des Trägers druckabfedernden Bereich (44) überlagern kann und sich der reibungsarme Bereich (70) in der Querrichtung (X) über beide Seiten des gegenüber dem Körper des Trägers druckabfedernden Bereichs (44) hinaus nach außen erstreckt.

2. Einweg-Urinkissen (1) gemäß Anspruch 1, wobei der reibungsarme Bereich (70) mindestens eines aus einem Siliziumharz und einem Fluorharz aufweist.

3. Einweg-Urinkissen (1) gemäß Anspruch 1 oder 2, wobei der reibungsarme Bereich (70) aus mehreren länglichen Unterbereichen (71) besteht, die sich in der Längsrichtung (Y) erstrecken und in der Querrichtung (X) diskontinuierlich angeordnet sind.

4. Einweg-Urinkissen (1) gemäß einem der Ansprüche 1 bis 3, wobei der reibungsarme Bereich (70) eine Basisschicht und ein Siliziumharz aufweist, das auf der Basisschicht verteilt ist.

5. Einweg-Urinkissen (1) gemäß einem der Ansprüche 1 bis 4, **dadurch gekennzeichnet, dass** das Einweg-Urinkissen eine erste und eine zweite Falzlinie (17, 18), die sich in der Querrichtung (X) erstrecken und in der Längsrichtung (Y) voneinander beabstandet sind, einen hinteren Bereich (16), der zwischen der hinteren Stirnkante (12) und der ersten Falzlinie (17) definiert ist, einen Zwischenbereich (14), der zwischen der ersten und der zweiten Falzlinie (17, 18) definiert ist, und einen vorderen Bereich (15) aufweist, der zwischen der zweiten Falzlinie (18) und der vorderen Stirnkante (11) definiert ist, wobei der reibungsarme Bereich (70) auf der die Haut nicht kontaktierenden Oberfläche des hinteren Bereichs (16) liegt und einen Reibungswiderstand hat, der kleiner als bei der Rückschicht (30) ist, und wobei der hintere Bereich (16) entlang der zweiten Falzlinie (17) auf den Zwischenbereich (14) gefaltet ist, sodass die die Haut kontaktierende Oberfläche des hinteren Bereichs (16) zur die Haut kontaktierenden Oberfläche des Zwischenbereichs (14) hin weist, und der vordere Bereich (15) entlang der ersten Falzlinie (18) auf die die Haut nicht kontaktierende Oberfläche des hinteren Bereichs (16) gefaltet ist, um so den reibungsarmen Bereich (70) abzudecken.

6. Einweg-Urinkissen (1) gemäß einem der Ansprüche 1 bis 4, **dadurch gekennzeichnet, dass** das Einweg-Urinkissen (1) eine erste und eine zweite Falzlinie (17, 18), die sich in der Querrichtung (X) erstrecken und voneinander in der Längsrichtung (Y) beabstandet sind, einen hinteren bereich (16), der zwischen der hinteren Stirnkante (12) und der ersten Falzlinie (17) definiert ist, einen Zwischenbereich (14), der zwischen der ersten und der zweiten Falzlinie (17, 18) definiert ist, und einen vorderen Bereich (15) aufweist, der zwischen der zweiten Falzlinie (18) und der vorderen Stirnkante (11) definiert ist, wobei der reibungsarme Bereich (70) auf der die Haut nicht kontaktierenden Oberfläche des hinteren Bereichs (16) liegt und einen Reibungswiderstand hat, der kleiner als bei der Rückschicht (30) ist, und wobei der vordere Bereich (15) entlang der zweiten Falzlinie (18) auf den Zwischenbereich (14) gefaltet ist, sodass die die Haut kontaktierende Oberfläche des vorderen Bereichs (15) zur die Haut kontaktierenden Oberfläche des Zwischenbereichs (14) hin weist, und der hintere Bereich (16) entlang der ersten Falzlinie (17) auf die die Haut nicht kontaktierende Oberfläche des vorderen Bereichs (15) gefaltet ist, um so den reibungsarmen Bereich (70) nach außen hin freizulegen.

## Revendications

1. Protection urinaire jetable (1) ayant une direction longitudinale (Y) et une direction transversale (X) et comprenant une surface pouvant être en contact avec la peau, une surface ne pouvant pas être en contact avec la peau s'étendant sur le côté opposé de la surface pouvant être en contact avec la peau, une ligne centrale transversale imaginaire (3-3) coupant une dimension de la protection urinaire dans la ligne centrale longitudinale (2-2), une direction longitudinale imaginaire (Y) coupant une dimension de la protection urinaire (1) dans la direction transversale (X), des bords d'extrémité avant et arrière (11, 12) s'étendant dans la direction transversale (X), une feuille supérieure (20) se trouvant sur la surface pouvant être en contact avec la peau, une feuille inférieure (30) se trouvant sur la surface ne pouvant pas être en contact avec la peau et un corps absorbant (40) se trouvant entre les feuilles supérieure et inférieure (20, 30), le corps absorbant (40) comprenant un matériau de coeur absorbant le liquide (41), et la surface ne pouvant pas être en contact avec la peau étant formée avec une région à faible friction (70) ayant une résistance à la friction inférieure aux autres régions et la région à faible friction (70) s'étendant entre la ligne centrale transversale imaginaire (3-3) et une région d'extrémité arrière (16) ;
**caractérisée en ce que** :
le corps absorbant (40) comprend une région d'amortissement de pression de corps (44) de l'utilisateur définie entre la ligne centrale transversale imaginaire (3-3) et le bord d'extrémité arrière (12) dans lequel une dimension dans le sens de l'épaisseur du matériau de coeur (41) est moins importante que dans les régions résiduelles de sorte que la région à faible friction (70) peut recouvrir la région d'amortissement de pression de corps (44) de l'utilisateur et la région à faible friction (70) s'étend vers l'extérieur dans la direction transversale (X) au-delà des deux côtés de la région d'amortissement de pression de corps (44) de l'utilisateur.

2. Protection urinaire jetable (1) selon la revendication 1, dans laquelle la région à faible friction (70) comprend au moins l'une parmi une résine de silicium et une résine fluorée.

3. Protection urinaire jetable (1) selon la revendication 1 ou 2, dans laquelle la région à faible friction (70) est composée d'une pluralité de sous-régions allongées (71) s'étendant dans la direction longitudinale (Y) et agencées de manière discontinue dans la direction transversale (X).

4. Protection urinaire jetable (1) selon l'une quelconque des revendications 1 à 3, dans laquelle la région à faible friction (70) comprend une feuille de base et une résine de silicium répartie sur la feuille de base.

5. Protection urinaire jetable (1) selon l'une quelconque des revendications 1 à 4, **caractérisée en ce que** la protection urinaire jetable comprend des première et seconde lignes de pliage (17, 18) s'étendant dans la direction transversale (X) et espacées l'une de l'autre dans la direction longitudinale (Y), une région arrière (16) définie entre le bord d'extrémité arrière (12) et la première ligne de pliage (17), une région intermédiaire (14) définie entre les première et seconde lignes de pliage (17, 18), une région avant (15) définie entre la seconde ligne de pliage (18) et le bord d'extrémité avant (11) et la région à faible friction (70) se trouvant sur la surface ne pouvant pas être en contact avec la peau de la région arrière (16) et ayant une résistance à la friction moins importante que dans la feuille inférieure (30), dans laquelle la région arrière (16) est pliée le long de la seconde ligne de pliage (17) sur la région intermédiaire (14) de sorte que la surface pouvant être en contact avec la peau de la région arrière (16) fait face à la surface pouvant être en contact avec la peau de la région intermédiaire (14) et la région avant (15) est pliée le long de la première ligne de pliage (18) sur la surface ne pouvant pas être en contact avec la peau de la région arrière (16) afin de recouvrir la région à faible friction (70).

6. Protection urinaire jetable (1) selon l'une quelconque des revendications 1 à 4, **caractérisée en ce que** la protection urinaire jetable (1) comprend des première et seconde lignes de pliage (17, 18) s'étendant dans la direction transversale (X) et espacées l'une de l'autre dans la direction longitudinale (Y), une région arrière (16) définie entre le bord d'extrémité arrière (12) et la première ligne de pliage (17), une région intermédiaire (14) définie entre les première et seconde lignes de pliage (17, 18), une région avant (15) définie entre la seconde ligne de pliage (18) et le bord d'extrémité avant (11) et la région à faible friction (70) se trouvant sur la surface ne pouvant pas être en contact avec la peau de la région arrière (16) et ayant une résistance à la friction moins importante que dans la feuille inférieure (30) et dans laquelle la région avant (15) est pliée le long de la seconde ligne de pliage (18) sur la région intermédiaire (14) de sorte que la surface pouvant être en contact avec la peau de la région avant (15) fait face à la surface pouvant être en contact avec la peau de la région intermédiaire (14) et la région arrière (16) est pliée le long de la première ligne de pliage (17) sur la surface ne pouvant pas être en contact avec la peau de la région avant (15) afin d'exposer la région à faible friction (70) à l'extérieur.
